# EUROPEAN PATENT APPLICATION

(11) **EP 1 593 405 A1**
(43) Date of publication of application: **09.11.2005**
(21) Application number: 03775918.0
(22) Date of filing: 27.11.2003
(51) Int. Cl.: A61M 39/14

(54) **JOINT DEVICE**

(30) Priority: 19.12.2002 JP 2002368715
(71) Applicant: KABUSHIKI KAISHA TOP, Adachi-ku, Tokyo 120-0035 (JP)
(72) Inventor: Suzuki, Hirohito, Kabushiki Kaisha Top, Adachi-ku, Tokyo 120-0035 (JP)
(74) Representative: Henkel, Feiler & Hänzel
(86) International application number: PCT/JP2003/015159
(87) International publication number: WO 2004/056417

(57) **Abstract**

A joint device is provided which can positively make a connection with a needle tube with a simple configuration when a syringe, a tube, and so on are connected. There are provided: an outer cylinder 8 which has a connecting part 11 for inserting and connecting a connected part 10 on its front end, a needle tube 5 which is stored in the outer cylinder 8 and is opened to the connecting part 11, and a closure member 13 which closes the needle tube 5 and freely moves forward and backward. The closure member 13 comprises a stopper 14 which is fit into the connecting part 11 having an inner periphery shaped in substantially a perfect circle, a needle tube contact part 15 making contact with the peripheral wall of the needle tube 5, and an urging part 16 for urging the stopper 14 in an advancing direction. The stopper 14 comprises a through hole 19 on a position opposed to an opening 6 of the needle tube 5. The stopper 14 has an oval outer periphery which is compressed in the major axis direction and is deformed in substantially a perfect circle so as to close the through hole 19 when the stopper 14 is fit into the connecting part.

## Description

### BACKGROUND OF THE INVENTION

### Field of the Invention

The present invention relates to a joint device and specifically relates to a joint device which is, for example, provided on a fluid flow path for intravenous drip and connects a syringe, a tube, and so on.

### Description of the Related Art

Conventionally this kind of joint device comprises a hollow needle tube extended inside a tubular outer cylinder and a cylindrical member which is inserted over the needle tube in the outer cylinder and is capable of sliding in the directions of moving forward and backward (e.g., Japanese Patent Laid-Open No. 10-15079).

The needle tube has a side hole opened perpendicularly to an axis on the peripheral wall near its closed end. The cylindrical member closes the side hole when being positioned on the end of the needle tube. Thus, when a luer tapered part or the like of a syringe is inserted to the front end of the outer cylinder, the cylindrical member is pressed by the cylindrical luer tapered part, which is provided on the front ends of the syringe, a tube, and so on, and is moved backward. The side hole of the needle tube is opened, and the needle tube simultaneously enters the luer tapered part, resulting in a passage state. Further, a coil spring is provided in the outer cylinder. The coil spring urges the cylindrical member, which is moved backward, in the advancing direction. Hence, when the luer tapered part is pulled out of the end of the outer cylinder, the cylindrical member is moved forward by the urging of the coil spring and the side hole of the needle tube is closed.

However, according to such a conventional configuration, when the luer tapered part is inserted into the front end of the outer cylinder, insufficient insertion reduces a retreating distance of the cylindrical member and the side hole of the needle tube may not be fully opened.

Further, when the side hole of the needle tube is not fully opened, a sufficient connection cannot be made and results in inconvenience. For example, a smooth fluid flow is hampered.

In order to eliminate such inconvenience, the present invention has as an object the provision of a joint device with a simple configuration which positively makes a connection with a needle tube when a syringe, a tube, and so on are connected.

### SUMMARY OF THE INVENTION

A joint device, comprising an outer cylinder shaped like a cylinder with a bottom, the outer cylinder having a connecting part opened so as to insert a connected part and make a connection with the connected part, a hollow needle tube which is extended along the axis of the outer cylinder, has a rear end connected outside the bottom of the outer cylinder, has a front end positioned a predetermined distance away from the connecting part in the outer cylinder, and is opened to the connecting part in the axial direction, and a closure member which is made of an elastic material, is stored in the outer cylinder so as to freely move forward and backward along the axial direction, and closes the outer cylinder and the needle tube on an advancing position, wherein the closure member comprises a stopper which is fit into the connecting part of the outer cylinder and tightly closes the connecting part, a cylindrical needle tube contact part which is connected to the rear end of the stopper and makes intimate contact with the peripheral wall of the needle tube, and an urging part for urging the stopper in the advancing direction via the needle tube contact part, the connecting part of the outer cylinder has an inner periphery shaped almost like a perfect circle, a through hole is formed on a position opposed to the opening of the needle tube in the stopper, the needle tube penetrating the through hole when the stopper moves backward, and the stopper has an oval outer periphery which is compressed in the major axis direction by the inner peripheral surface of the connecting part and is deformed almost like a perfect circle coincident with the connecting part so as to close the through hole when the stopper is fit into the connecting part.

According to the joint device of the present invention, for example when the connected part such as a luer tapered part provided on a syringe and a tube is inserted to the connecting part of the outer cylinder, the stopper of the closure member is pressured and moved backward. Accordingly, the needle tube penetrates the stopper via the through hole formed on the stopper. Since the front end of the needle tube is opened in the axial direction, the closure is released on the opening of the front end of the needle tube when the front end of the needle tube penetrates the stopper, and a connection is made with the syringe and the tube. In this way, the joint device of the present invention can positively open the opening on the front end of the needle tube when the front end of the needle tube penetrates the stopper and can prevent insufficient opening on the opening of the needle tube, as compared with a conventional configuration in which a cylindrical member opens and closes a side hole opened perpendicularly to the axis of a needle tube.

Moreover, when the luer tapered part is pulled out of the connecting part of the outer cylinder, the stopper is moved forward by the urging force of the urging part. Thus, the needle tube is pulled out of the through hole of the stopper and the stopper is fit into the connecting part.

Incidentally the front end of the needle tube is opened in the axial direction. On the stopper for closing the opening, the through hole is formed so as to be opposed to the opening of the needle tube. Thus, when a fluid or the like flowing via the opening of the needle tube reaches the through hole, there is a probability that the fluid or the like may break the closure of the through hole and leak to the outside of the outer cylinder.

Hence, in the present invention, the stopper has an oval outer periphery which is fit into the connecting part having an inner periphery shaped in substantially a perfect circle. Thus, the stopper is deformed in substantially a perfect circle according to the shape of the connecting part and then the through hole can be closed by pressing in the major axis direction of the stopper. Therefore, even when the through hole is formed so as to be opposed to the opening of the needle tube, it is possible to positively prevent a leak of a fluid or the like from the through hole.

Further, the present invention is characterized in that the through hole of the stopper is opened and formed like an oval figure having a major axis coincident with the minor axis direction of the stopper. The stopper having the oval outer periphery is pressed in the major axis direction by the inner peripheral surface of the connecting part when being fit into the connecting part. On the other hand, since the through hole has a major axis coincident with the minor axis direction of the stopper, edges opposed in the minor axis direction are brought into contact with each other when the stopper is fit into the connecting part. Thus, the through hole is quickly closed when the stopper is fit into the connecting part and the closure of the through hole can be firmly maintained by a pressing force applied in the major axis direction of the stopper from the connecting part.

At this point, on the side of the stopper that is opposed to the opening of the needle tube, a protrusion protruding almost like a circular cone is formed which is gradually reduced in diameter toward the needle tube at least in a predetermined range including the through hole when the stopper is fit into the connecting part. On the inner periphery of the outer cylinder, an inclined part is formed which is inclined while being gradually increased in diameter along the retreating direction of the stopper between the connecting part and the front end of the needle tube. The inclined part opens the through hole on the tip of the protrusion while guiding and sliding the stopper so as to restore the stopper to an oval figure when the stopper is moved backward.

When the stopper closes the connecting part of the outer cylinder, a fluid may reversely flow to the stopper through the needle tube and enter between the closure member and the needle tube. In this case, since the protrusion is provided on the inner surface of the stopper that is opposed to the needle tube, a fluid having reached the protrusion flows along the outer periphery of the protrusion and disperses a hydraulic pressure, thereby positively preventing a leak from the through hole.

Moreover, when the protrusion is provided, while a leak is prevented, the tip of the protrusion may hit the opening on the front end of the needle tube and prevent insertion of the needle tube into the through hole when the stopper is moved backward. Thus, the inclined part is provided on the inner periphery of the outer cylinder and the retreating stopper is guided by the inclined part. The stopper guided by the inclined part is increased in diameter along the inclined part from the side opposed to the needle tube. Accordingly the through hole formed on the protrusion is opened so as to receive the needle tube from a part opposed to the needle tube, the stopper is further moved backward, and thus the needle tube can be smoothly inserted into the tube through hole.

The urging part is shaped almost like a conical cylinder which is connected to the rear end of the needle tube contact part, is extended along the needle tube, is gradually separated from the needle tube toward the rear end of the needle tube, and comprises a plurality of ring-shaped thin parts. The thin parts can be bent are arranged at predetermined intervals along the axial direction. When the stopper is moved backward along the needle tube, the urging part is bent via the thin parts, the small diameter side is stored inside the large diameter side, and an urging force is applied to the stopper by the restoring elasticity of the urging part.

Since the urging part is connected to the rear end of the needle tube contact part, the number of components is reduced and assembling efficiency is improved. Further, when the urging part is compressed and an urging force is generated by restoring elasticity, the urging part is bent via the ring-shaped thin parts, the small diameter side is stored inside the large diameter side, and the total length of the urging part can be relatively short during compression. Thus, it is possible to achieve a small space for storing the urging part in the outer cylinder and achieve a compact configuration for the joint device.

### BRIEF DESCRIPTION OF THE DRAWINGS

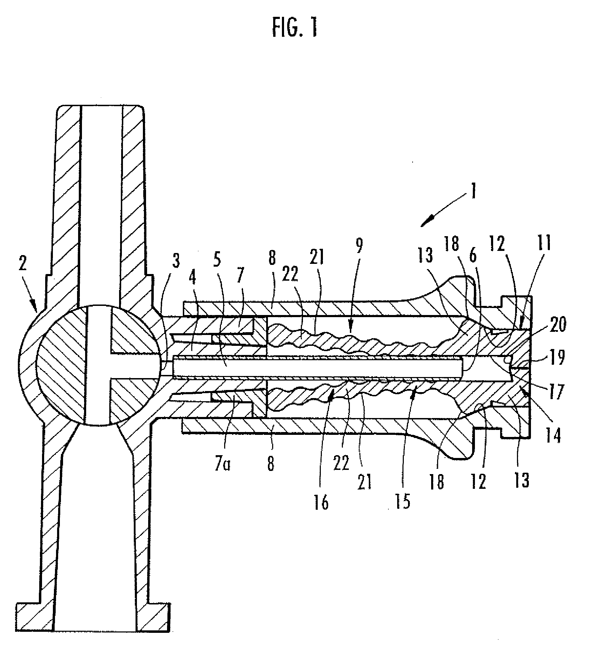

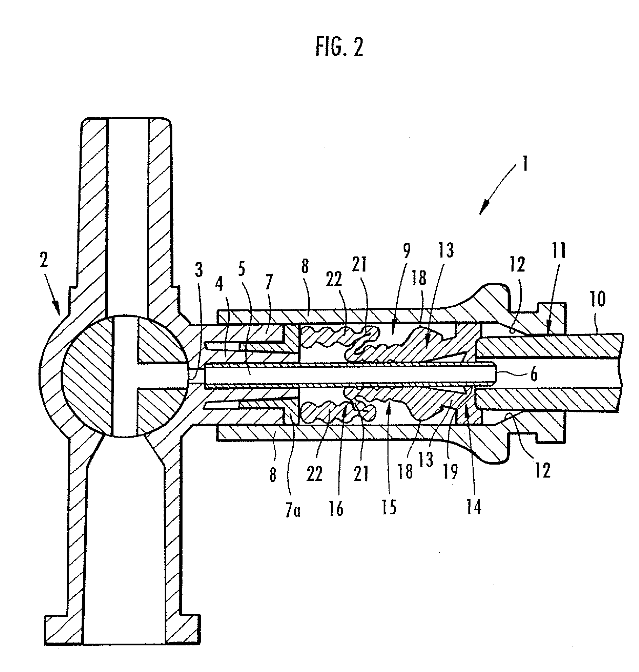

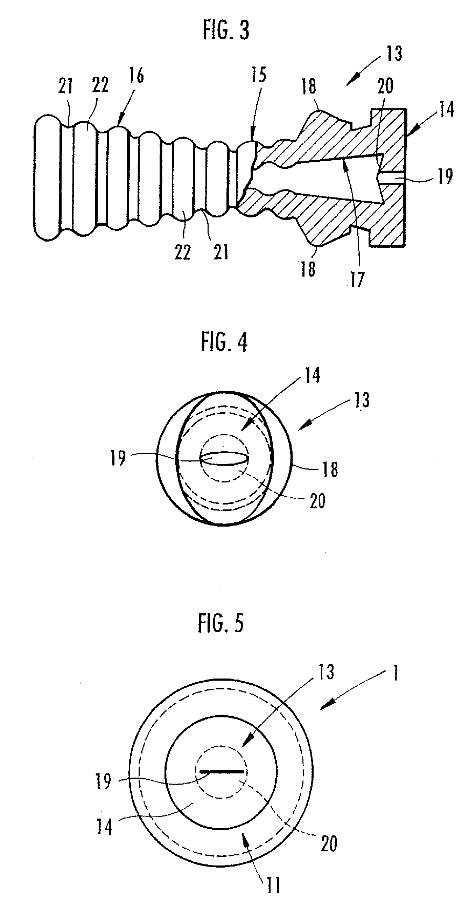

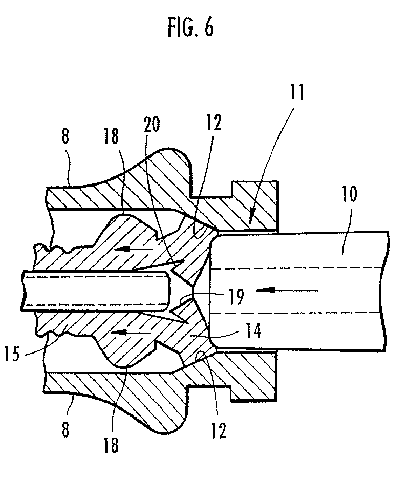

FIG. 1 is an explanatory sectional view showing a joint device of the present embodiment, FIG. 2 is an explanatory sectional view showing a connecting state of the joint device shown in FIG. 2, FIG. 3 is an explanatory sectional view showing a closure member, FIG. 4 is a front view showing the closure member, FIG. 5 is a front view showing the joint device shown in FIG. 1 and FIG. 6 is an explanatory sectional view showing an operation of the main part of the closure member.

### DETAILED DESCRIPTION OF THE PREFERRED EMBODIMENTS

The following will describe an embodiment of the present invention in accordance with the accompanying drawings.

As shown in FIG. 1, the joint device 1 of the present embodiment is provided on a port 3 of a three way stop cock 2. A metal needle tube 5 is inserted to the port 3 via a cylindrical needle tube supporting part 4. The needle tube 5 has a base end connected to the port 3. An opening 6 opened in the axial direction is formed on the front end of the needle tube 5.

As shown in FIGS. 1 and 2, an outer cylinder 8 is connected and supported on the outer periphery of the needle tube supporting part 4 via a cylindrical outer cylinder supporting part 7. In the outer cylinder 8, a needle tube storing part 9 is formed to store the needle tube 5 up to its front end. Ahead of the front end of the needle tube 5 stored in the needle tube storing part 9 in the outer cylinder 8, a connecting part 11 is formed with an inner periphery shaped in substantially a perfect circle so that a cylindrical luer tapered part 10 (FIG. 2), which is provided on the ends of a syringe and a tube (not shown), can be inserted into the connecting part 11. The connecting part 11 is smaller in inside diameter than the needle tube storing part 9. On an inner wall between the connecting part 11 and the needle tube storing part 9, an inclined part 12 is formed which gradually increases in inside diameter from the connecting part 11 to the needle tube storing part 9.

Further, a closure member 13 made of an elastic material such as a synthetic rubber is stored in the outer cylinder 8. As shown in FIGS. 1 and 3, the closure member 13 integrally comprises a stopper 14 tightly fit into the connecting part 11 of the outer cylinder 8, a needle tube contact part 15 tightly making contact with the peripheral wall of the needle tube 5, and an urging part 16 for urging the stopper 14 in the advancing direction when the stopper 14 is pulled out of the connecting part 11 of the outer cylinder 8 and is moved backward. The urging part 16 has a rear end making contact with a contact member 7a which is fit into the outer cylinder supporting part 7 and efficiently generates an urging force during expansion and retraction. Moreover, a connecting part 17 for integrally connecting the stopper 14 and the needle tube contact part 15 is provided between the stopper 14 and the needle tube contact part 15. A slip-off preventing protrusion 18 protruding to the outer periphery is formed on the connecting part 17.

As shown in FIG. 4, the stopper 14 has an outer periphery shaped in an oval figure. The oval figure of the stopper 14 has a major axis longer than the inside diameter of the connecting part 11. Further, at the center of the stopper 14 (as shown in FIG. 1, a position opposed to the needle tube 5), a through hole 19 is formed which is opened in an oval figure. The oval figure of the through hole 19 is in a state so as to have a major axis coincident with the minor axis direction of the stopper 14. Thus, as shown in FIG. 5, the stopper 14 fit into the connecting part 11 is pressed to the inner peripheral surface of the connecting part 11 of the outer cylinder 8 and is deformed in substantially a perfect circle. Accordingly the through hole 19 is closed elastically.

Further, as shown in FIGS. 1 and 3, a protrusion 20 protruding in a circular cone to the needle tube 5 is formed on the inner surface of the stopper 14. As shown in FIG. 1, the protrusion 20 is formed on a part where the through hole 19 is provided. The tip of the protrusion 20 is opposed to the front end of the needle tube 5.

As shown in FIGS. 1 and 3, the urging part 16 extends to the rear end of the needle 5 while being connected to the needle tube contact part 15. The urging part 16 is shaped substantially a conical cylinder which is gradually separated from the needle tube 5 along the direction toward the rear end of the needle tube 5. Moreover, the urging part 16 is formed so that a plurality of ring-shaped thin parts 21 and a plurality of ring-shaped thick parts 22 are arranged alternately. As shown in FIG. 2, the urging part 16 can be bent by providing the thin parts 21. At this point, the urging part 16 is not only compressed but also bent via any one of the thin parts 21 so that the smaller diameter part of the urging part 16 is stored inside the large diameter part thereof. An urging force generated by the restoring elasticity of the urging part 16 is applied to the needle tube contact part 15 and the stopper 14. At this point, since the total length of the urging part 16 is relatively short during compression, a small storage space for the urging part 16 in the outer cylinder 8 can be achieved. Additionally, the urging part 16 is not limited to such a configuration. For example, the following configuration is applicable (not shown): the thin parts 21 and the thick parts 22 are not provided or a metal coil spring is provided to apply an urging force from the compressed coil spring to the needle tube contact part 15 and the stopper 14. In the present embodiment, as shown in FIG. 3, the urging part 16 is integrally connected to the needle contact part 15. Thus, as compared with the use of the coil spring, the number of components is smaller and assembling efficiency is improved.

In the joint device 1 of the present embodiment configured thus, as shown in FIG. 1, the outer cylinder 8 and the needle tube 5 are closed while the stopper 14 is fit into the connecting part 11 of the outer cylinder 8. At this point, the stopper 14 shaped in an oval figure is pressed in themaj or axis direction by the inner peripheral surface of the connecting part 11 of the outer cylinder 8 and the stopper 14 is deformed in substantially a perfect circle coincident with the opening shape of the connecting part 11 of the outer cylinder 8. Hence, the through hole 19 is firmly closed. Moreover, the needle tube contact part 15 is brought into intimate contact with the outer periphery of the needle tube 5, preventing a leak to the outside of the closure member 13. Additionally, on the position of the through hole 19, the protrusion 20 shaped in a circular cone is formed which has a tip protruding inward, achieving the same effect as a so-called check valve. Thus, it is possible to more positively prevent an inside hydraulic pressure from opening the through hole 19. Further, the slip-off preventing protrusion 18 protruding to the outer periphery of the connecting part 17 makes contact with the inclined part 12 of the outer cylinder 8 and prevents the stopper 14 from slipping out of the outer cylinder 8. Additionally, in the present invention, the stopper 14 shaped in an oval figure is deformed in substantially a perfect circle coincident with the opening shape of the connecting part 11 of the outer cylinder 8, and firmly closes the through hole 19. Thus, even in the case of a flat surface not having the conical protrusion 20, it is possible to sufficiently prevent the opening of the through hole 19.

As shown in FIG. 2, in the joint device 1, when the luer tapered part 10 is inserted into the connecting part 11, the needle tube 5 is inserted into the luer tapered part 10 and permits the passage of a drug solution. When the luer tapered part 10 is inserted into the connecting part 11, the joint device 1 operates as follows: when the luer tapered part 10 is inserted into the connecting part 11, the leading edge of the luer tapered part 10 is pressed and contacted to the stopper 14, and the luer tapered part 10 and the stopper 14 make tight contact with each other. Simultaneously the stopper 14 is pressed by the luer tapered part 10 and is moved backward. Accordingly, the stopper 14 firstly moves out of the connecting part 11, slides along the inclined part 12, and is deformed along the inclined part 12. In the deforming state of the stopper 14, as show in FIG. 6, the outer diameter is increased from the inner surface side. Since the through hole 19 is formed on the conical protrusion 20, the tip of the protrusion 20 is expanded and opened so as to open the inside edges of the through hole 19.

When the stopper 14 further moves backward, as shown in FIG. 2, the needle tube 5 is relatively inserted into the opened through hole 19 and the stopper 14 enters the needle storing part 9. In this way, even when the protruding part 20 is provided, the needle tube 5 can smoothly penetrate the through hole 19 without any trouble. Further, the needle tube 5 having penetrated the through hole 19 is inserted into the luer tapered part 10.

Meanwhile, the urging part 16 is deformed and bent via the thin parts 21 and generates relatively high restoring elasticity when the stopper is moved backward, and an urging force is applied to the stopper 14 in the advancing direction. Thus, the contact state of the luer tapered part 10 and the stopper 14 is maintained and the outer periphery of the needle tube 5 maintains the intimate contact with the needle tube contact part 15, thereby positively preventing a leak in a connecting state.

Further, as shown in FIG. 2, when the joint device 1 and the luer taperedpart 10 are connectedto each other, the stopper 14 is urged in the advancing direction by the urging part 16. Hence, only by pulling the luer tapered part 10 out of the connecting part 11, the stopper 14 is fit into the connecting part 11 and the outer cylinder 8 and the needle tube 5 are closed as shown in FIG. 1.

### INDUSTRIAL APPLICABILITY

The present invention can be mounted on a fluid tube for medical treatment and enables a syringe to be positively connected when another solution is infused by the syringe to a fluid flow path for intravenous drip, achieving a smooth medical operation.

## Claims

1. A joint device, comprising:
an outer cylinder in cylindrical shape with a bottom, the outer cylinder having a connecting part opened so as to insert a connected part and make a connection with the connected part,
a hollow needle tube which is extended along an axis of the outer cylinder, has a rear end connected outside a bottom of the outer cylinder, has a front end positioned a predetermined distance away from the connecting part in the outer cylinder, and opened to the connecting part in an axial direction, and
a closure member which is made of an elastic material, is stored in the outer cylinder so as to freely move forward and backward along the axial direction, and closes the outer cylinder and the needle tube on an advancing position,
wherein the closure member comprises a stopper which is fit into the connecting part of the outer cylinder and tightly closes the connecting part, a cylindrical needle tube contact part which is connected to a rear end of the stopper and makes intimate contact with a peripheral wall of the needle tube, and an urging part for urging the stopper in an advancing direction via the needle tube contact part,
the connecting part of the outer cylinder has an inner periphery shaped in substantially a perfect circle,
a through hole is formed on a position opposed to an opening of the needle tube in the stopper, the needle tube penetrating the through hole when the stopper moves backward, and
the stopper has an oval outer periphery which is compressed in a major axis direction by an inner peripheral surface of the connecting part and is deformed in substantially a perfect circle coincident with the connecting part so as to close the through hole when the stopper is fit into the connecting part.

2. The joint device according to claim 1, wherein the through hole of the stopper is opened and formed in an oval figure having a major axis coincident with a minor axis direction of the stopper.

3. The joint device according to claim 2, wherein on a side of the stopper that is opposed to the opening of the needle tube, a protrusion protruding substantially in a circular cone is formed which is gradually reduced in diameter toward the needle tube at least in a predetermined range including the through hole when the stopper is fit into the connecting part,
on an inner periphery of the outer cylinder, an inclined part is formed which is inclined while being gradually increased in diameter along a retreating direction of the stopper between the connecting part and the front end of the needle tube, and
the inclined part opens the through hole on a tip of the protrusion while guiding and sliding the stopper so as to restore the stopper to an oval figure when the stopper is moved backward.

4. The joint device according to any one of claims 1 to 3,
wherein the urging part is shaped in substantially a conical cylinder which is connected to a rear end of the needle tube contact part, is cylindrically extended along the needle tube, is gradually separated from the needle tube toward the rear end of the needle tube, and comprises a plurality of ring-shaped thin parts, the thin parts being bent and arranged at a predetermined interval along the axial direction, and
when the stopper is moved backward along the needle tube, the urging part is bent via the thin parts, a small diameter side is stored inside a large diameter side, and an urging force is applied to the stopper by restoring elasticity of the urging part.
